(19) Europäisches Patentamt
European Patent Office
Office européen des brevets

(11) **EP 2 181 691 B1**

(12) **EUROPEAN PATENT SPECIFICATION**

(45) Date of publication and mention
of the grant of the patent:
**21.12.2016 Bulletin 2016/51**

(51) Int Cl.:
*A61K 8/65* *(2006.01)*      *A61K 8/73* *(2006.01)*
*A61K 8/37* *(2006.01)*      *A61K 8/11* *(2006.01)*
*A61Q 15/00* *(2006.01)*

(21) Application number: **08167667.8**

(22) Date of filing: **27.10.2008**

(54) **Antiperspirant compositions**

Schweißhemmende Zusammensetzungen

Compositions anti-transpirantes

(84) Designated Contracting States:
**AT BE BG CH CY CZ DE DK EE ES FI FR GB GR
HR HU IE IS IT LI LT LU LV MC MT NL NO PL PT
RO SE SI SK TR**

(43) Date of publication of application:
**05.05.2010 Bulletin 2010/18**

(73) Proprietors:
• **Unilever PLC**
**London**
**EC4Y 0DY (GB)**
Designated Contracting States:
**CY GB IE MT**
• **Unilever N.V.**
**3013 AL Rotterdam (NL)**
Designated Contracting States:
**BE BG CH CZ DE DK EE ES FI FR GR HR HU IS
IT LI LT LU LV MC NL NO PL PT RO SE SI SK TR**

(72) Inventors:
• **Chan, Catrin, Sian**
**Leeds, LS14 2AR (GB)**

• **Cropper, Martin, Peter**
**Wirral, Merseyside, CH63 3JW (GB)**
• **Franklin, Kevin, Ronald**
**Wirral, Merseyside, CH63 3JW (GB)**
• **Johnson, Simon, Anthony**
**Wirral, Merseyside, CH63 3JW (GB)**
• **McKeown, Robert**
**Wirral, Merseyside, CH63 3JW (GB)**

(74) Representative: **Whaley, Christopher**
**Unilever Patent Group**
**Colworth House**
**Sharnbrook**
**Bedford MK44 1LQ (GB)**

(56) References cited:
**EP-A- 0 385 534      EP-A- 0 480 520**
**EP-A- 0 519 531      WO-A-2006/056096**
**FR-A- 2 839 658      US-A- 5 043 161**

**Description**

The present invention relates to antiperspirant compositions and more particularly to anhydrous compositions.

During the course of normal day to day activities, such as from physical activity or from being subjected to stress, mankind perspires, resulting, in the absence of remedial treatment, in damp patches on the skin or in clothing that comes into contact with such skin and in the generation of malodour arising from skin microflora acting upon secretions from inter alia eccrine glands. The distribution of eccrine glands is uneven in the skin, being concentrated particularly in the underarm. Consequently, the underarm is where damp patches and/or malodour generation is most prone to occur.

In many societies, body malodour is considered to be undesirable or even anti-social. Similarly, persons exhibiting damp patches can suffer embarrassment or even criticism. As a consequence, an industry has grown up to prevent or at least hinder sweating and to reduce the generation of body malodour by the creation of cosmetic antiperspirant compositions that are applied topically to skin, particularly in those localised parts of the body where the density of eccrine glands is greatest, such as in the underarm. Whilst bathing can remove sweat, it is not preventative and in any event, there is growing pressure on the world's water so that regular use of antiperspirants as part of a cleanliness regime helps to conserve water supplies. Moreover, the cost of heating water by gas boilers or by electricity generated from gas has increased substantially since 2000, so that regular bathing or showering in an increasingly expensive activity. Commonly, body malodour is counteracted by both washing and regular application of an antiperspirant composition.

The antiperspirant industry has recognised that it is convenient for users to apply an antiperspirant composition after washing, commonly in the bathroom or shower-room such as once or twice a day, for example in the morning or evening, or during preparation for a social gathering. It is less convenient to apply antiperspirant at work or during a social gathering, so that it would be desirable for the effectiveness of an antiperspirant composition to last for several hours, such as up to 24 hours after application.

It would also be reassuring for the user to be reminded periodically of the continuing effectiveness of the antiperspirant composition, removing the anxiety that itself could induce sweating. One of the means adopted by the industry to reduce the impact of malodour generation on the body is the incorporation of a fragrance or a plurality of fragrances into the antiperspirant composition. The fragrance can mask the body odour and some fragrances, so-called deo-fragrances, can also function as a bactericide or bacteristat preventing or retarding the formation of malodorous compounds on the skin.

It has long since been understood that a fragrance functions by being evaporated from the skin onto which it has been applied topically, so that after a while, its concentration falls below that at which it is olfactorily detected. The rate at which that will occur depends on the volatility of the perfume component and its inherent volatility, often approximated by its boiling point. The art of the perfumer is to select a blend of perfume components that satisfies the aesthetic criteria for that perfume. However, it has also been recognised that the perfumer can slow down the rate of evaporation of a perfume by encapsulating it, thereby prolonging the effective life of the perfume after it has been applied topically. Likewise, it was recognised that it was necessary for the perfume to be released from the capsules containing it. In other words, the encapsulation has to be reversible.

A number of encapsulating materials have been proposed for encapsulating perfumes, including modified starches and various gums, including acacia gum. Such materials are water soluble at body temperature, so that the disruption of the capsule wall can be triggered by moisture, including, in particular, sweat. Whilst this has the advantage of being localised and triggered by a sweat event, it has the disadvantage that sweat, on production, is typically substantially odourless, whereas malodour is generated over an extended period of time by the action of localised microflora, such as Coryne bacteria transforming solutes within the excretion. Thus, the masking perfume can be released before the malodour is generated and there is a significant risk that it will have been dissipated before the malodour is fully generated. Accordingly, it would be desirable to employ an alternative encapsulating material, one that does not rely upon sweating to cause its wall to rupture.

Anhydrous contact compositions comprise some of the commonest types of antiperspirant composition, and especially sticks (firm solids) or creams (soft solids) in North America. Anhydrous antiperspirants are characterised by the absence of an aqueous liquid phase. Contact compositions are characterised by being applicable directly from a dispensing container into direct contact with skin. In other regions, roll-on compositions are popular contact compositions, and yet elsewhere aerosol or other spray compositions are the most widely sold compositions, in which the dispenser is held at a short distance, eg 15 cms, from the skin surface and the composition is sprayed onto the skin.

Many antiperspirant salts, such as aluminium and/or zirconium halohydrate salts comprise a small fraction of bound water, possibly in the region of from 1 to 3% by weight of the antiperspirant salt, but such bound water is present within the solid salt particles and not as "free" water. Accordingly, compositions containing them are still deemed to be anhydrous. It would be particularly desirable to be able to devise anhydrous antiperspirant compositions containing an encapsulated perfume in which the capsule wall was disrupted by a means other than sweat.

It has been proposed to incorporate encapsulated perfume into aqueous fabric or hair conditioning compositions employing an encapsulating material that is water-insoluble, and shear sensitive. The capsules are incorporated as an aqueous slurry and the resultant composition applied to a substrate, via an aqueous composition. Subsequently, the fragrance is released by the substrate being rubbed, or abraded, typically quite vigorously, thereby rupturing the shell of the encapsulate. The presence of water has often been considered to be of crucial importance, because the rupture-resistance of the capsule wall (the shell) is sensitive to moisture. In an aqueous medium, the shear-sensitive encapsulates are much more resistant to being ruptured. When the capsules are dry, their resistance is significantly diminished.

It has been found during the course of research leading to the instant invention that powdery shear sensitive encapsulates containing a fragrance can be suspended effectively in a carrier liquid for the particulate antiperspirant active material comprising a hydrophobic liquid, commonly called an oil or a mixture of oils, in anhydrous antiperspirant compositions, provided that the encapsulates satisfy certain physical criteria. In such circumstances, a significant fraction of the encapsulates remains intact and is capable of releasing its contents after application of the antiperspirant composition onto skin, such as in the armpit. The perfume can then be released by lateral movement of clothing or other skin across and in contact with the surface of skin to which the composition was applied, or by impact of for example the arm on the chest during normal day-to-day movement of the arm.

If the encapsulates have physical characteristics outside defined ranges, pre-application retention or post-application release of fragrance is impaired. For example, if the capsules are too weak, they do not survive the manufacture process or the application process, whereas if they are too strong, greater pressure is needed to rupture them than is provided by one area of skin or clothing rubbing past the area of skin to which the composition has been applied or by normal impact of arm on chest.

Brief summary of the present invention

According to a first aspect of the present invention, there is provided an anhydrous antiperspirant composition characterised by the absence of an aqueous phase comprising a particulate antiperspirant active,
a dry particulate shear sensitive encapsulated perfume,
a liquid carrier for the particulate antiperspirant active and shear sensitive encapsulated perfume comprising at least one water-immiscible oil
in which the particulate encapsulated perfume comprises capsules having:-

> a shell of cross-linked gelatin coacervate,
> a volume average particle diameter in the range of 25 to 70 $\mu$m,
> a shell having a measured thickness in the range of from 0.25 to 9 $\mu$m,
> a ratio of the shell thickness to the average particle diameter in the range of from 1:5 to 1:120,
> and a Hysitron hardness, measured in a Hysitron Tribo-indenter fitted with a Berkovich tip and programmed to perform an indent by compressing a sample with an initial contact force of 75 $\mu$N, for 10 seconds, followed by a position hold stage for 1 second and a decompression stage for 10 seconds, in the range of from 2 MPa to 50 MPa, blended with a propellant for application from a pressurised aerosol dispenser.

By the employment of such a dry particulate shear sensitive encapsulated perfume satisfying selected characteristics, it is possible to deposit on skin a residual fraction of shear-sensitive encapsulated perfume particles that can be ruptured by the passage of a garment across the surface of the skin or by the movement of one area of skin relative to another, such as in the underarm, at a time when sweating is or is not occurring or irrespective of whether sweating has occurred. Advantage is accordingly taken of the sensitivity of such a dry particle on the skin surface to be ruptured by relative movement of garment or skin to skin, but the presence of the thickened or gelled liquid carrier enables the significant fraction of the capsules to be so deposited from conventional contact applicators or from conventional aerosol dispensers. This enables improved masking of malodour and enhanced perception of fragrance over a prolonged period.

Although it is possible for some encapsulates having characteristics outside the ranges identified herein to offer some residual fragrance release activity, for example encapsulates having a shell formed from starches or related water-soluble or dispersible materials, when triggered by encountering sweat, the selection of encapsulates satisfying the specified ranges according to the present invention combines manufacturing capability under the conditions for making anhydrous stick antiperspirant compositions with greater availability of releasable fragrance in the underarm.

The antiperspirant composition of the present invention may be used for simultaneously a) preventing or reducing localised sweating by topical application of a composition according to the first aspect and b) prolonging perception of perfume or masks body-generated malodour, even when sweating is not occurring or irrespective of whether sweating has occurred. Detailed Description of the invention, including preferred embodiments.

The present invention relates to the incorporation into anhydrous antiperspirant compositions of shear sensitive encapsulated perfume capsules, the term capsules herein including microcapsules. Shear sensitive herein contemplates that the capsule is capable of releasing its perfume contents by rubbing of the upper arm forwards or backwards across in contact with the chest wall whilst remaining in contact with it or by the rubbing of clothing worn on the upper arm or chest likewise rubbing across skin in the upper arm or armpit to which the antiperspirant composition has been applied.

The encapsulating material for the shear-sensitive capsules herein is cross-linked gelatin. One encapsulation process suitable for forming shear sensitive capsules is often called complex coacervation, which has been described, for example, in USP6045835. In such a process, an aqueous solution of a cationic polymer, commonly gelatin or a closely related cationic polymer, is formed at an elevated temperature that is high enough to dissolve the gelatin, commonly at least 40° and in many instances it is unnecessary to exceed 70°C. A range of 40 to 60°C is very convenient. The solution is typically dilute, often falling in the range of from 1 to 10% w/w and particularly from 2 to 5% w/w. Either before or after dissolution of the gelatin, an oil-in-water emulsion is formed by the introduction of a perfume oil, optionally together with a diluent oil if desired.

A polyanion or like negatively charged polymer is introduced and the composition diluted until a pH is attained of below the isoelectric point of the system, such as below pH5, and particular from pH3.5 to pH 4.5, whereupon a complex coacervate forms around the dispersed perfume oil droplets. The polyanion commonly comprises gum arabic or a charged carboxymethyl cellulose derivative, such an alkali metal salt, of which sodium is the most commonly mentioned example.

The resultant shell is subsequently cross linked, with a short chain aliphatic di-aldehyde, for example a $C_4$ to $C_6$ dialdehyde, including in particular glutaraldehyde. The cross linking step is commonly conducted at a temperature of below ambient such as from 5 to 15°C, and particularly in the region of 10°C. Representative weights and proportions of the reactants and of suitable operating conditions are shown in Examples 1, 2 or 3 of the aforementioned US 6045835. The skilled man by suitable selection of the parameters within the general process outlined therein is well capable of producing capsules having a volume average particle size in the range of from 30 to 100$\mu$m, particularly up to 75$\mu$m and especially 40 to 60$\mu$m.

A second encapsulation method that is likewise suitable for forming encapsulated perfumes in which the shell comprises cross-linked coacervated gelatin comprises variations of the above process, as contemplated in WO2006/056096. In such variations, microcapsules comprising a blank hydrogel shell are first formed in a dry state and brought into contact with an aqueous or aqueous/alcoholic mixture of a fragrance compound, commonly diluted with a diluent oil. The fragrance compound is transported through the hydrogel shell by aqueous diffusion and is retained inside. The resultant fragrance-containing microcapsules are then dried to a powder, which for practical purposes is anhydrous. Although selection of the ratio of fragrance oil to diluent oil is at the discretion of the producer, and may be varied over a wide range, the ratio is often selected in the range of from 1:2 to 1:1, and particularly 3:4 to 1:1, for fragrance : diluent oils.

The proportion of shell material to core perfume oil is at the discretion of the producer, and is attainable by appropriately varying the proportions of the ingredients in the emulsion. It is desirable for the shell material to constitute from 10 to 80% of the capsules, and particularly from 10 to 40% by weight and especially from 12 to 25% by weight of the capsules. By varying the proportions of shell and core, the physical strength of the shell can be varied (for capsules of the same volume average particle size). Accordingly, capsules having the desired combination of characteristics can be selected.

In some preferred embodiments of the present invention, the fragrance oil constitutes from 70 to 85% by weight of the encapsulates and in such embodiments, the balance is provided by the shell.

In other preferred embodiments, the fragrance oil is present together with an oil diluent, for example providing from 25 to 75% by weight of the oil mixture held within the shell, and especially from 40 to 60% by weight. Desirably in such embodiments, the shell constitutes from 12 to 25% by weight of the encapsulates. In certain of such preferred embodiments, the fragrance constitutes from 35 to 50% by weight of the encapsulates, and is complemented by 35 to 50% by weight of diluent oil. If desired, in yet other embodiments, the composition contains some of the encapsulates that contain diluent oil and others that do not, the weight ratio of the two sets of encapsulates being selected in the range of from 25:1 to 1:25 at the discretion of the producer.

It is preferred for the volume average particle diameter (size) of the capsules to be at least 40 $\mu$m and in many desirable embodiments is up to 60 $\mu$m in diameter. Herein, unless otherwise indicated, the volume average particle diameter of the encapsulates (D[4,3]) is that obtainable using a Malvern Mastersizer, the encapsulates being dispersed in cyclopentasiloxane (DC245) using a dispersion module mixer speed of 2100 rpm. Calculations are made using the General Purpose model, assuming a spherical particle shape and at Normal calculation sensitivity. The shell thickness can be measured by solidifying a dispersion of the capsules in a translucent oil, cutting a thin slice of the solid mass and using a scanning electron microscope to obtain an image of cut-through individual capsules, thereby revealing the inner and outer outline of its annular shell and hence its thickness.

The shell thickness of the microcapsules tend to increase as the particle size increases. The shell thickness accordingly, often ranges mainly within the thickness range of from 0.25 to 9$\mu$m and for many desirable capsules having shells made from coacervated gelatin, at least 90% by volume of the capsules have shells of up to 2.5 $\mu$m thickness. Desirably, at least 95% by volume3 of the capsules have a shell thickness of at least 0.25 $\mu$m. The average shell thickness of microcapsules desirably employed herein is up to 1.5 $\mu$m. The same or other suitable gelatin coacervate capsules have an average shell thickness of at least 0.4 $\mu$m. For capsules of diameter up to 40 $\mu$m, the shell thickness is often below 0.75 $\mu$m, such as from 0.25 to <0.75$\mu$m whereas for particle of at least 40 $\mu$m the shell thickness is often from 0.6 to 2.5 $\mu$m.

The fragrance-containing capsules for incorporation in the anhydrous antiperspirant compositions are commonly selected having a ratio of volume average diameter:average shell thickness in the range of from 10:1 to 100:1 and in many desirable such capsules in the range of from 30:1 or 40:1 to 80:1.

By virtue of the particle size and the shell thickness of the capsules, the average % volume of the core containing the fragrance oils and any diluent oil, if present, often falls within the range of from 50 to 90%, and in many embodiments from 70 to 87.5%.

The hardness of the capsules, as measured in a Hysitron Tribo-indenter, is an important characteristic that enables them to be incorporated effectively in anhydrous formulations, retaining the capability of being sheared by frictional contact between skin and skin or clothing. The hardness is desirably in the range of from 0.5 to 50 MPa and especially from 2.5 or 5 up to 25 MPa, and in many embodiments is up to 10 MPa. In certain preferred embodiments, the hardness is in the range of from 3.5 to 5.5 MPa.

A further parameter of interest in relation to the capsules in the instant invention, and particularly their capability to be sheared by friction in the compositions and process of the instant invention, is their "Apparent Reduced Elastic Modulus (Er). Desirably, Er falls within the range of from 20 to 35 MPa, and in many convenient embodiments, in the range of from 22 to 30MPa.

The hardness (Hysitron Hardness) and Apparent Reduced Elastic Modulus herein are those measured by the following method:-

A drop of a dispersion of the capsules in demineralised water is placed onto a piece of silicon wafer and allowed to dry leaving behind discrete micro encapsulates for mechanical analysis. The dried wafer is fitted into the Hysitron Tribo-indenter, and spatially mapped using the optical system of the instrument to identify a perimeter around the sample.

The head of the Tribo-indenter is fitted with a Berkovich tip, a three sided pyramid, which compresses individual capsules. A single capsule is positioned directly under the Indenter tip. The instrument is programmed to perform an indent by compressing the sample with an initial contact force of 75$\mu$N, for 10 seconds, followed by a position hold stage for 1 second and a decompression stage for 10 seconds. The instrument achieves a very small load (typically around 15-30 $\mu$N). The Hysitron Hardness (H in MPa) and reduced Elastic Modulus (Er in MPa) are calculated from the relaxation stage of the force deflection data using the following equations.

$$H = \frac{W}{A}$$

W = Compressive force
A = Contact Area ($A \approx 24.56 h_c^2$)

$$Er = \frac{\sqrt{\pi}}{2\gamma} \frac{S}{\sqrt{A}}$$

S = Contact Stiffness ($dW/dh_t$)
$h_t$ = Total Penetration Depth
$\gamma$ = 1.034

$$h_c = h_t - \kappa \frac{W}{S}$$

$\kappa$ = 3/4
$h_c$ = Contact Depth

Results are averages of a minimum of 20 measurements made on capsules with a particle size of D[4,3] +/- 20%

By control of the manufacturing process conditions, the resultant dry capsules having the characteristics specified in the ranges or preferred ranges for particles size and mean diameter described herein can be obtained.

The capsules, by virtue of their manufacture route often contain a small residual water content. It is desirable, for example, as measured by the conventional Karl Fischer method, to select capsules having a residual water content of below 5% by weight and particularly below 4% by weight, such as from 0.5 to 3.5% and particularly from 0.6 to 3% w/w (based on the fragrance-containing capsule). Based on the weight of the shell, said water content of the capsules employed herein often falls in the range of from 1% to 20% w/w. By limiting the proportion of water in the capsule, and particularly in the shell, it is possible to avoid at least partly, and preferably substantially, the formation of grit within the anhydrous formulation, and thereby avoid the negative sensation of grit on underarm skin. Grit occurs typically when particles aggregate to form agglomerates that are not readily fractured into their constituent particles. Accordingly, in regard to aerosol or spray compositions, the avoidance of grit formation has a second benefit of reducing the likelihood of blockage of the spray nozzle.

The shear sensitive encapsulate or mixture of encapsulates can be employed in the antiperspirant compositions in an amount at the discretion of the manufacturer. Commonly, the amount is at least 0.05%, in many instances at least 0.1% and often at least 0.3% by weight of the composition. Usually, the amount is up to 5%, desirably up to 4% and in many instances is up to 3% by weight of the composition. A convenient range is from 0.5 to 2.5% by weight of the composition. Accordingly, the base compositions before introduction of propellant contain a proportionately higher proportion of the encapsulate.

The perfume oil employable herein in the shear sensitives capsules, and/or other capsules and/or non-encapsulated can be selected as is conventional to attain the desired aesthetic result, and comprises usually a blend of at least 5 components, and often at least 20 components. The components can be synthetic or natural extractions, and, in the case of natural oils or oils produced to mimic natural oils, are often mixtures of individual perfume compounds. The perfume oil can comprise, inter alia, any compound or mixture of any two or more such compounds coded as an odour (2) in the Compilation of Odor and Taste Threshold Values Data edited by F A Fazzalari and published by the American Society for Testing and Materials in 1978.

Often, though not exclusively, the perfume compounds acting as perfume components or ingredients in blends have a ClogP (octanol/water partition coefficient) of at least 0.5 and many a ClogP of at least 1. Many of the perfume components that are employable herein can comprise organic compounds having an odour that is discernible by humans that are selected within the chemical classes of aldehydes, ketones, alcohols, esters, terpenes, nitriles and pyrazines. Mixtures of compounds within classes or from more than one class can be blended together to achieve the desired fragrance effect, employing the skill and expertise of the perfumer. As is well known, within the same class, those compounds having a lower molecular weight, often up to about 200, tend to have a lower boiling point and be classified as "top notes", whereas those having a higher molecular weight tend to have a higher boiling point and be classified as middle or base notes. The distinction, though, is to some extent an arbitrary simplification, because the fragrance oils form a continuum and their characteristics are not significantly different close to on either side of an arbitrary boundary such as a boiling point of 250°C or 275°C. Herein, the perfume can comprise any blend of oils boiling at below 250°C (such as in the range 1 to 99% or 4 to 96%, 10 to 90% or 25 to 60%) with the balance provided by compounds having a boiling point above 250°C. The perfumer recognises that the lower boiling point compounds tend to evaporate more quickly after exposure, whereas higher boiling point compounds tend to evaporate more slowly, so that the desired aesthetic effect can be achieved by selecting the proportions of the faster and slower compounds - the faster providing an instant "hit" whilst the slower providing a longer lasting impact. It will also be recognised that a term such as high impact has also been used to describe low boiling point perfume compounds. The properties of the compound stay the same irrespective of whether they are called high impact or top note ingredients.

A further characteristic of a perfume compound is its odour detection threshold (ODT). Some perfume oils are much more easily detected by the human nose than others, but it is a very subjective measurement and varies considerably depending on the way that testing is performed, the prevailing conditions and the make-up of the panel, e.g. age, gender and ethnicity. As a qualitative means of differentiating between the aesthetic attributes of compounds, and enabling the perfumer to choose ingredients that are detected relatively easily, the ODT represents a useful guide, but quantitatively is more dubious.

Examples of perfume compounds or oils that can be employed within the capsules described herein, ether singly, or more usually as a blend of at least 5 or at least 10 or at least 20 or at least 50 compounds listed hereinafter include:-1,1,6,7- tetramethyl naphthalene, 1-(2,6,6-trimethyl-1,3-cyclohexandienyl)-2-buten-1-one, 1-(5,5-dimethyl-1-cyclohexen-1-yl)-1h-indene-a-propanal, 1,2,3,4,5,6,7,8-octahydro-1,1,6,7-tetramethyl naphthalene, 1,3-oxathiane, 11-dodecatetraenal, 1-methyl-4-isopropenyl-1-cyclohexene, 2,3-dihydro-1,1-dimethyl-(9ci), 2,4-dimethyl 2,6,-nonenol, 2,4-dimethylcyclohexene-3-carbaldehyde, 2-n-heptyl cyclopentanone, 2,6-nonadienal, 2,6-dimethyl-2,6-octadien-8-ol, 2,6-dimethyl-5-heptenal, 2,6-nonadien-1-ol, 2-isobutylthiazole, 2-methoxy-3- (2-methylpropyl)- pyrazine, 2-methoxy-4-(2-propenyl)phenol, 2-methoxy-4-vinylphenol, 2-methyl-4-propyl-cis-paradiff, 2-propenyl ester, 3- methyl-4-(2,6,6- trimethyl-2- cyclohexen-1-yl) cyclogalbanate, 3-(3-isopropylphenyl)butanal, 3,3-dimethyl-5-(2,2,3-trimethyl-3-cycloenten-1-yl)-4-penten-2-ol, 3,6 nonadienol, 3,7-dimethyl-1,6-octadien-3-ol, 3,7-dimethyl-2, 3,7-dimethyl-2,6-octadienenitrile, 3,7-dimethyl-6-octen-1-ol, 3-buten-2-one, 3-cyclohexadienyl)-3-buten-4-one, 3-cyclohexene-1-carboxaldehyde, 3-methyl-2-buten-1-yl acetate, 3-methyl-4-(2,6,6-trimethyl-2-cyclohexen-1-yl), 3-p-cumenyl-propionaldehyde 4-(1-methylethyl)benzenepropanal, 4 dodecenal, 4-(2,6,6-trimethyl-2-cyclohexen-1-yl)(e), 4-methoxybenzaldehyde, 4-methyl-3-decen-5-ol, 4-methyl-4-mercaptopentan-2-one, 4-penten-1-one, 6-(z,3-pentenyl)-tetrahydro-(2h)-pyranone-2, and 3-methyl-(cis-2-penten-1-yl)-2-cyclopenten-1-one, 6-octadienal, 6-trimethyl-1, 7-acetyl 1 2 3 4 5 6 7 8- octahydro-1, 8-cyclohexadecen-1-one, acetic acid (cyclohexyloxy,2-propenyl ester, acetyl cedrene, acetyl tributly citrateadoxal, aldehyde supra, allyl amyl glycolate, allyl caproate, allyl cyclohexane propionate, allyl heptanoate, allyl heptoate, alpha damascone, alpha methyl ionone iso, alpha pinene, alpha terpineol, alpha thujone, ambrox, ambroxan, amyl acetate, amyl cinnamic aldehyde, amyl salicylate, and isopropyl quinoline, anethol, anisic aldehyde, applinate, aurantiol, bacdanol, benzaldehyde citral polysantol, benzyl acetate, benzyl alcohol, benzyl salicylate, beta gamma hexenol, beta naphthol methyl ether, beta pinene, borneol, bornyl acetate, buccoxime, butyl anthranilate, calone, calone 1951, camphor, carvacrol, carvone, cashmeran, cedramber, cetalox, cinnamic alcohol, cinnamic aldehyde, cis 1,3-oxathiane-2-methyl-4-propyl, cis 3 hexenyl acetate, cis-3-hexenyl acetate, cis-3-hexenyl salicylate, cis-6 nonenol, citral, citronellal nitrile, citronellol, citronellyl acetate, citronellyl oxyacetaldehyde, clonal, corps cassis 0.1% tec, coumarin, cyclal c, cyclemax, cyclo galbanate, cymal, damascene, damascenone, damascone alpha, damascone beta, decyl alcohol, decyl aldehyde, delphone, delta damascone, diacetyl, dihydro iso jasmonate, dihydro myrcenol, dimethyl benzyl carbinol, dimethyl benzyl carbinyl butyrate, dimethyl benzyl carbinyl isobutyrate, d-limonene, ebanol, ethyl 2 4 decadienoate, ethyl 2 methyl butyrate, ethyl aceto acetate, ethyl anthranilate, ethyl butyrate, ethyl caproate, ethyl maltol, ethyl methyl dioxolane acetate, ethyl methyl phenyl glycidate, ethyl vanillin, ethyl-2-4-decadienoate, ethyl-2-methyl- butyrate, eucalyptol, eugenol, exaltolide/cyclopentadecanolide, excital, flor acetate, floralozone, florhydral, fructone, frutene, furaneol, galaxolide, galbex, gamma decalactone, gamma dodecalactone, gamma nonalactone, gamma undecalactone, geraniol, geranyl acetate, geranyl nitrile, geranyl phenylacetate, grapefruit zest (ca), habanolide, helional, heliotropin, heptylcyclopentanone, herbavert, hexahydro-4,7 methano-1h-inden-5(or 6)-yl propionate, hexanoic acid, hexyl acetate, hexyl cinnamic aldehyde, hexyl salicylate, hivemal, hydroquinone dimethyl ether, hydroxycitronellal, indol, intreleven aldehyde, ionone alpha, ionone beta, ionone gamma, irisantheme, iso 2-methoxy-4-(2-propenyl)phenol, isobutyl benzoate, isobutyl cinnamate, iso cyclo citral, iso e super, iso eugenol, iso eugenol acetate, isolongifolanone, iso propyl quinoline, iso-amyl acetate, iso-amyl alcohol, iso methylionone, isononyl acetate, isononyl formate, isovaleric aldehyde 0.1% dpg, javanol, keone, kharismal, labienoxime, lauric aldehyde, lemon juice carbonyls, lilial, liminal, limonene, linalool, linalyl acetate, linalyl benzoate, linalyl cinnamate, lyral, maltol, mandarin aldehyde, manzanate, maple lactone, melonal, menthol, methyl anthranilate, methyl beta naphthyl ketone, methyl cedrylone, methyl cedrylone 0.1041 84.44 811, methyl dihydro jasmonate, methyl dihydrojasmonate, methyl heptine carbonate, methyl isobutenyl tetrahydro pyran, methyl nonyl acetaldehyde, methyl nonyl ketone, methyl octine carbonate, methyl phenyl carbinyl acetate, methyl phenyl carbonyl acetate, methyl-3,4-dioxy(cylcoacetonyl) benzene, nectaryl, neobutanone, nerol, nirvanol, nonalactone, nonanediol-1,3-diacetate, nonanolide-1,4, nonyl aldehyde, nonyl formate, norlimbanol, octyl aldehyde, orange juice carbonyls, orange sinensal, ortho tertiary butyl cyclohexanyl acetate, oxane, oxocyclohexadecen-2-one, ozonil, p.t. bucinal, p-1-menthen-8thiol, para cresol, para cresyl methyl ether, para cymene, para hydroxy phenyl butanone, para hydroxyl phenyl butanone, paradiff, paramenthene, patchouli, pentalide, phenoxy ethyl isobutyrate, phenoxy ethyl propionate, phenyl acetaldehyde, phenyl acetaldehyde dimethyl acetal, phenyl ethyl alcohol, phenyl ethyl dimethyl carbinol, phenyl propyl alcohol, pinoacetaldehyde, polysantol, prenyl acetate, pyrazines, pyrazobutyle, pyridine acetyl 10%, rhubofix, sandalore, sandalore, sandela, spirogalbone, sulfurol, tangerinal, tetra hydro 3,7-dimethyl-1,6-octadien-3-ol, tetrahydrolinalool, tetrahydro muguol, thymol, trans 4 decenal, trans-2-hexenal, tricyclo decenyl acetate, tridecene-2-nitrile, triethyl citrate, trifemal, triplal, undecalactone, undecavertol, undecyl aldehyde, undecylenic aldehyde, vanillin, veloutone, veloutone, verdox, violiff, yara yara, zingerone.

Some of such perfume raw materials have a boiling point of less than, or equal to, 250° C, including some which are generally known to have a low odour detection threshold. Others within said list of perfume raw materials have a boiling point of greater than 250°C of which some are also generally known to have a low odour detection threshold.

Alternatively or additionally, the fragrance incorporated into the capsules can comprise one or a mixture of perfume essential oils, either mixed with each or and/or with synthetic analogues and/or one or more individual perfume compounds, possibly extracted from blossom, leaves, seeds fruit or other plant material. Oils which are herein contemplated include oils from:-

Bergamot, cedar atlas, cedar wood, clove, geranium, guaiacwood, jasmine, lavender, lemongrass, lily of the valley, lime, neroli, musk, orange blossom, patchouli, peach blossom, petotgrain, pimento, rose, rosemary, and thyme.

It will be recognised that since naturally derived oils comprise a blend in themselves of many components, and the perfume oil commonly comprises a blend of a plurality of synthetic or natural perfume compounds, the perfume oil itself in the encapsulate does not exhibit a single boiling point, ClogP or ODT, even though each individual compound present therein does.

If desired, the composition can include one or more perfume ingredients that provide an additional function beyond smelling attractively. This additional function can comprise deodorancy. Various essential oils and perfume ingredients, for example those passing a deodorant value test as described in US 4278658 provide deodorancy as well as malodour masking.

In the invention described herein, the carrier oil in which the capsules (and the antiperspirant active) are suspended comprises one or more oils, by which is meant liquids that are water-immiscible. Such oils are characterised by being liquid at 20°C (at 1 atmosphere pressure) and are often selected from silicone oils, hydrocarbon oils, ester oils, ether oils and alcohol oils or a mixture of two or more oils selected from such classes of oils. It is highly desirable that the oil has a boiling point of above 100°C and preferably above 150°C.

One class of oils that is highly favoured comprises volatile silicone oils, which often contribute from 20% to 95% by weight of a blend of oils, particularly at least 30% and in many convenient blends at least 40% by weight. It is advantageous in the instant invention to employ a blend in which the weight proportion of the volatile silicone oils is up to 80% by weight, and particularly up to 70% by weight. The balance of the oils in the blend is provided by one or more non-volatile silicone oils and/or one or more of the other classes of oils.

Herein, a volatile silicone oil is a liquid polyorganosiloxane having a measurable vapour pressure at 25°C of at least 1 Pa, and typically in a range of from 1 or 10 Pa to 2kPa. Volatile polyorganosiloxanes can be linear or cyclic or mixtures thereof. Preferred cyclic siloxanes, otherwise often referred to as cyclomethicones, include polydimethylsiloxanes and particularly those containing from 3 to 9 silicon atoms, preferably at least 4 and especially at least 5 silicon atoms. Preferred cyclomethicones contain not more than 7 silicon atoms and very preferably up to 6 silicon atoms. Volatile silicone oils herein desirably contain on weight average from 4.5 to 5.9 silicone atoms, and especially at least 4.9.

Preferred linear polyorganosiloxanes include polydimethylsiloxanes containing from 3 to 9 silicon atoms. The volatile siloxanes normally by themselves exhibit viscosities of below $10^{-5}$ m$^2$/sec (10 centistokes), and particularly above $10^{-7}$ m$^2$/sec (0.1 centistoke), the linear siloxanes normally exhibiting a viscosity of below 5 x $10^{-6}$ m$^2$/sec (5 centistokes). The volatile silicones can also comprise linear or cyclic siloxanes such as the aforementioned linear or cyclic siloxanes substituted by one or more pendant -0-Si(CH$_3$)$_3$ groups, the resultant compounds desirably containing not more than 7 silicon atoms.
Examples of commercially available silicone oils include oils having grade designations 344, 345, 244, 245 and 246 from Dow Corning Corporation; Silicone 7207 and Silicone 7158 from Union Carbide Corporation; and SF1202 from General Electric.

Highly desirably, the compositions according to the present invention comprise either an ether oil or an ester oil or both, preferably in a proportion of greater than 10% w/w of the composition, and particularly greater than 20% w/w. Although together, they could constitute up to 100% w/w of the carrier oils blend, it is desirable that together they contribute no greater than 60% w/w and in many compositions, they total up to 50% w/w of the blend.

The ester oils can be aliphatic or aromatic. Suitable aliphatic ester oils comprise at least one residue containing from 10 to 26 carbon atoms and a second residue of at least 3 carbon atoms up to 26 carbon atoms. The esters may be mono or diesters, and in the latter be derived from a C$_3$ to C$_8$ diol or di carboxylic acid. Examples of such oils include isopropyl myristate, isopropyl palmitate, myristyl myristate.

It is especially desirable to employ an aromatic ester, including especially benzoate esters. Preferred benzoate esters satisfy the formula Ph-CO-O-R in which R is:-an aliphatic group containing at least 8 carbons, and particularly from 10 to 20 carbons such as from 12 to 15, including a mixture thereof;
or an aromatic group of formula -A-Y-Ph in which A represents a linear or branched alkylene group containing from 1 to 4 carbons and Y represents an optional oxygen atom or carboxyl group.
Particularly preferably, the aromatic ester comprises C$_{12-15}$ alkyl benzoate.

The ether oil preferably comprises a short chain alkyl ether of a polypropylene glygol (PPG), the alkyl group comprising from C2 to C6, and especially C4 and the PPG moiety comprising from 10 to 20 and particularly 14 to 18 propylene glycol units. An especially preferred ether oil bears the INCI name PPG14-butyl ether.

The ester and ether oils herein are selected having a boiling point in excess of 100°C. This enables them to be employed with all wax systems for solidifying the carrier oil that typically melt at no higher than 95°C, and commonly between 65 and 85°C. For sticks made using small molecule gelling agents, it is preferable to select oils having a boiling point in excess of 150°C, and they, naturally, are suitable in conjunction with wax systems too.

The ester and ether oils can be present in the composition in a weight ratio to each other of from 1:0 to 0:1, and in some embodiments from 10:1 to 1:10.

Indeed, though such oils have a number of other beneficial properties, such as for example, reducing the extent to which the antiperspirant formulation is visible after application on the skin, compositions in which the oil blend contains only a minor as compared with a major proportion of such ether and ester oils tend to exhibit sensory attributes preferred by many consumers. In practice, it is desirable for greater than 5% by weight of the oil blend, especially greater than 10% and especially greater than 15% by weight of the oil blend to be furnished by the ester and ether oils. The combined weight of the two oils is preferably less than 60%, particularly less than 50% and especially less than 40% of the weight of the oil blend.

The carrier oil blend can further comprise one or more other water-immiscible oils that have a melting point of below 20°C and a boiling point of above 100°C and preferably above 150°C, including hydrocarbon oils, including preferably non-volatile hydrocarbon oils, non-volatile silicone oils and aliphatic monohydric alcohols.

In the instant invention, non-volatile oils, sometimes referred to as emollient oils, such as non-volatile silicone or/and hydrocarbon oils can desirably be included to alter the sensory attributes of the compositions containing, such as to soften the skin or to assist in masking the visibility of particulate materials deposited on the skin. However, it is desirable to restrict the proportion of such non-volatile oils to less than 30% by weight of the oil blend, and in various compositions according to the instant application, the total proportion of such oils is from 5 to 20% by weight.

Examples of suitable non-volatile hydrocarbon oils include polyisobutene and hydrogenated polydecene. Examples of suitable non-volatile silicone oils include dimethicones and linear alkylarylsiloxanes. The dimethicones typically have an intermediate chain length, such as from 20 to 100 silicon atoms. The alkylarylsiloxanes are particularly those containing from 2 to 4 silicon atoms and at least one phenyl substituent per silicon atom, or at least one diphenylene group. The aliphatic alcohol desirably is a branched chain monohydric alcohol containing from 12 to 40 carbon atoms, and often from 14 to 30 carbon atoms such as isostearyl alcohol.

One further class of ester oils that can constitute a fraction of the ester oils contemplated in the invention compositions comprises natural plant oils, commonly containing glyceride esters and in particular the glyceride triesters of unsaturated C18 aliphatic carboxylic acids, such as linoleic acid, linolenic acid or ricinoleic acid, including isomers such as linolenelaidic acid, trans 7-octadecenoic acid, parinaric acid, pinolenic acid punicic acid, petroselenic acid, columbinic acid and stearidonic acid. Examples of such beneficial natural oils include caster oil, coriander seed oil, impatiens balsimina seed oil, parinarium laurinarium kernel fat, sabastiana brasilinensis seed oil borage seed oil, evening primrose oil, aquilegia vulgaris oil, for and sunflower oil and safflower oil. Such oils can desirably comprise from 1 to 10% by weight of the oil blend.

The weight of fragrance materials is not included herein in calculating the weight of the oil blend, irrespective of whether the fragrance is encapsulated or "free".

Antiperspirant Actives

The composition contains an antiperspirant active. Antiperspirant actives are preferably incorporated in an amount of from 0.5-50%, particularly from 5 to 30% and especially from 10% to 26% of the weight of the composition. It is often considered that the main benefit from incorporating of up to 5% of an antiperspirant active in a stick composition is manifest in reducing body odour, and that as the proportion of antiperspirant active increases, so the efficacy of that composition at controlling perspiration increases.

Antiperspirant actives for use herein are often selected from astringent active salts, including in particular aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates.

Aluminium halohydrates are usually defined by the general formula $Al_2(OH)_xQ_y.wH_2O$ in which Q represents chlorine, bromine or iodine, x is variable from 2 to 5 and x + y = 6 while $wH_2O$ represents a variable amount of hydration. Especially effective aluminium halohydrate salts, known as activated aluminium chlorohydrates, are described in EP-A-6739 (Unilever NV et al), the contents of which specification is incorporated herein by reference. Such activated aluminium chlorohydrates are made by a method in which the weight concentration of aluminium compounds in the solution is controlled within specified limits and simultaneously the temperature of that solution is controlled within a specified elevated temperature range whilst polymeric aluminium species are formed, and drying conditions are strictly controlled as described in the said EP-A-6739. Some activated salts do not retain their enhanced activity in the presence of water but are useful in substantially anhydrous formulations, i.e. formulations that do not contain a distinct aqueous phase.

Zirconium actives can usually be represented by the empirical general formula: $ZrO(OH)_{2n-nz}B_z.wH_2O$ in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by $wH_2O$. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-$\beta$-phenylalanine, dl-valine, dl-methionine and $\beta$-alanine, and preferably glycine which has the formula $CH_2(NH_2)COOH$.

It is highly desirable to employ complexes of a combination of aluminium halohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from B K Giulini, from Summit and from Reheis, though with differing particle size distributions.

Many aluminium and/or zirconium-containing astringent antiperspirant salts employed herein have metal: chloride mole ratio in the range of 1.3:1 to 1.5:1. Others having a lower metal:chloride mole ratio, such as from 1:1 to 1.25:1 tend to generate lower pHs when applied to skin and thus tend to be more irritating.

The proportion of solid antiperspirant salt in a suspension composition normally includes the weight of any water of hydration and any complexing agent that may also be present in the solid active.

Many particulate antiperspirants employed in the instant invention have a refractive index (RI) of at least 1.49 and not higher than 1.57. Actives which are free from zirconium tend to have an RI of from 1.49 to 1.54, depending on their formula and at least partly on their residual water content. Likewise, actives which contain zirconium tend to have an RI of from 1.52 to 1.57.

The selection of the antiperspirant active material desirably takes into account the type of applicator from which it is dispensed. In embodiments in which the composition is dispensed as a spray, such as using an aerosol dispenser, the antiperspirant active is highly desirably an aluminium chlorohydrate (ACH) or an activated aluminium chlorohydrate (AACH).

For incorporation of compositions according to the present invention, desirably at least 90%, preferably at least 95% and especially at least 99% by weight of the particles having a diameter in the range of from 0.1$\mu$m up to 100$\mu$m.

For incorporation in non-contact applicators and especially in aerosols in which the composition is expelled from the dispenser by a propellant gas, possibly augmented by a mechanical or electromechanical propulsive means, it is especially desirable for less than 5% by weight, particularly less than 1% by weight and advantageously none of the particles to have a diameter of below 10$\mu$m. Preferably for inclusion in aerosol compositions, the particles have a diameter of below 75$\mu$m. In many preferred aerosol compositions, the antiperspirant has an average ($D_{50}$) particle diameter in the range of from 15 to 25$\mu$m. The particle size of the antiperspirant active or mixture of actives can be measured using a Malvern Mastersizer, similarly to measurement of the perfume microcapsules size, as mentioned hereinbefore.

One method of seeking to minimise visible whiteness employs antiperspirant active material that is free or substantially free from hollow particles. In this context, substantially free indicates a content of less than 10% by weight hollow spheres, and preferably less than 5% by weight. Some drying techniques, eg spray drying, can produce materials which contain greater than such a proportion of hollow spheres, the proportion can be reduced by milling the particulate material, such as by ball or swing milling.

In embodiments for application from a pressurized aerosol dispenser, the anhydrous composition, deemed to be a base composition, and desirably incorporating a suspension aid, is blended with a propellant.

The anhydrous compositions can contain one or more optional ingredients, such as one or more of those selected from those identified below.

Optional ingredients include wash-off agents, often present in an amount of up to 10% w/w to assist in the removal of the formulation from skin or clothing. Such wash-off agents are typically nonionic surfactants such as esters or ethers containing a $C_8$ to $C_{22}$ alkyl moiety and a hydrophilic moiety which can comprise a polyoxyalkylene group (POE or POP) and/or a polyol.

The compositions herein can incorporate one or more cosmetic adjuncts conventionally contemplatable for cosmetic solids or soft solids. Such cosmetic adjuncts can include skin feel improvers, such as talc or finely divided (i.e. high molecular weight) polyethylene, i.e. not a wax, for example Accumist™, in an amount of 1 up to about 10%; a moisturiser, such as glycerol or polyethylene glycol (mol wt 200 to 600), for example in an amount of up to about 5%; skin benefit agents such as allantoin or lipids, for example in an amount of up to 5%; colours; skin cooling agents other than the already mentioned alcohols, such a menthol and menthol derivatives, often in an amount of up to 2%, all of these percentages being by weight of the composition. A further optional ingredient comprises a preservative, such as ethyl or methyl paraben or BHT (butyl hydroxy toluene) such as in an amount of from 0.01 to 0.1% w/w.

Aerosol base compositions desirably additionally comprise a suspending aid, sometimes called a bulking agent which is typically a powdered silica or a layered clay, such as a hectorite, bentonite or montmorillonite. The layered clay is optionally hydrophobically surface treated. Particularly suitable surface treated clays are available under the trade mark Bentone, such as Bentone 38. The suspending aid often constitutes from 0.5 to 4% by weight of the base aerosol composition. Aerosol base compositions desirably also can contain a swelling aid to assist swelling of the layered clay, often selected in a proportion of from 0.005 to 0.5% by weight of the aerosol base composition and particularly in a weight ratio to the clay of from 1:10 to 1:75. Suitable swelling aids include especially propylene carbonate and triethyl citrate.

The invention compositions herein can additionally contain a water-soluble polymer comprising a Bronsted acid group that cooperates synergistically with the aluminium or aluminium/zirconium antiperspirant active to enhance antiperspirant efficacy. Such a material is referred to in US6616921 as a co-gellant (because it assists the antiperspirant active to gel in eccrine pores) and is described therein. Preferred examples of such a co-gellant are polymers having a molecular weight of at least 50,000 derived at least in part from maleic acid or maleic anhydride, such as Gantraz™ AN119, AN139 or AN169. The co-gellant is often selected in a weight ratio to the aluminium or aluminium/zirconium salt of from 1:15 to 1:2.

The invention compositions herein can additionally comprise a non-encapsulated fragrance, for example in a weight % of from 0.01 to 4% of the composition, and particularly from 0.1 to 1.5%. The non-encapsulate fragrance is desirably incorporated into the composition in a weight ratio to the shear-sensitive encapsulate in the range of from 5:1 to 1:5. The non-encapsulated fragrance can be created from the same palette of perfume materials described above. The non-encapsulated fragrance can, if desired, be the same as or similar to the encapsulated fragrance, but it is often more attractive if the two fragrances are different, because this minimises the extent to which the nose has become desensitised to perfume. Choice of the various fragrances and the differences between them, such as proportion of top notes, is primarily a matter of aesthetic judgement.

Additionally or alternatively to the non-encapsulated fragrance, if desired the compositions herein can comprise fragrance encapsulated in a water-sensitive shell, such that when a person sweats, the aqueous excretion ruptures the shell, releasing fragrance. Such water-sensitive encapsulates are described for example in EP0303461. Additionally or likewise alternatively, the compositions herein can comprise a cyclic oligosaccharide such as cyclodextrins, including $\alpha$ or $\beta$ cyclodextrin, each optionally substituted by a methyl or hydroxy-propyl group that associates reversibly with free fragrance. Such materials are described in EP1289484. The composition can contain the water-sensitive fragrance encapsulate and/or cyclic oligosaccharide in an amount of from 0.1% to 4% by weight of the composition.

The weight ratio of shear-sensitive encapsulate to water-sensitive encapsulate and/or cyclic oligosaccharide is often selected in the range of from 5:1 to 1:5.

The invention compositions desirably are substantially or totally free from water-soluble short chain monohydric alcohols (commonly recognised as up to $C_6$) and especially ethanol. Substantially in this context indicates a proportion of less than 5% and preferably less than 1% by weight of the respective full or base composition.

Herein unless the context demands otherwise, all weights, %s, and other numbers can be qualified by the term "about".

Aerosol products herein comprise a base composition comprising an antiperspirant and/or deodorant active suspended in an oil blend together with the fragrance capsules, suspending agent and optional ingredients that is blended with a propellant, commonly in a weight ratio of blend to propellant of from 1:1 to 1:15, and in many formulations from 1:3 to 1:9. The propellant is commonly either a compressed gas or a material that boils at below ambient temperature, preferably at below 0°C, and especially at below -10°C. Examples of compressed gasses include nitrogen and carbon dioxide. Examples of low boiling point materials include dimethylether, $C_3$ to $C_6$ alkanes, including in particular propane, butanes and isobutane, optionally further containing a fraction of pentane or isopentane, or especially for use in the USA the propellant is selected from hydrofluorocarbons containing from 2 to 4 carbons, at least one hydrogen and 3 to 7 fluoro atoms.

Aerosol products can be made in a conventional manner by first preparing a base composition, charging the composition into the aerosol can, optionally introducing the fragrance into the can after the base composition, (late fill addition), fitting a valve assembly into the mouth of the can, thereby sealing the latter, and thereafter charging the propellant into the can to a desired pressure, and finally fitting an actuator on or over the valve assembly together with an overcap if the can does not employ through the cap spraying.

Product Dispenser

A suitable dispenser for an aerosol composition comprises a can, usually made from steel or aluminium, often having a coated interior to prevent contact between the can contents and the can wall, which contents can be vented to the exterior through a dip tube leading via a valve that is openable and closable by an actuator, into a spray channel terminating in a spray nozzle. Suitable dispensers are described, for example in EP1219547, EP1255682, or EP1749759.

Having summarised the invention and described it in more detail, together with preferences, specific embodiments will now be described more fully by way of example only.

The capsules E1 and E2 employed herein comprised a shell made from a complex coacervate of gelatin with respectively gum Arabic or carboxymethylcellulose, cross linked with glutaraldehyde. E1 is in accordance with the process of WO2006/056096 and E2 in accordance with US6045835, in each instance with conditions controlled to obtain the specified shell wall and capsule hardness characteristics identified in Table 1 below.

Table 1

| Characteristic | Capsules E1 | Capsules E2 |
|---|---|---|
| Mean particle size D[4,3] | 48.4 $\mu$m | 50.7 $\mu$m |
| Shell thickness (19 to 38$\mu$m) | 0.3-0.65 $\mu$m | |
| Shell thickness (25 to 35$\mu$m) | | 0.25-0.6 $\mu$m |
| Shell thickness calculated at mean particle size | 1.0-1.6 $\mu$m | 1.4-2.2 $\mu$m |
| DR (11 to 18$\mu$m) | 40:1 - 58:1 | 60:1 - 100:1 |
| DR (calculated shell thickness) | 30:1 - 48:1 | 23:1 - 36:1 |
| Hysitron hardness | 4.05 MPa | 4.88MPa |
| Apparent Reduced Elastic Modulus | 24.1 MPa | 27.5 MPa |
| Wt % oils/fragrance in core | 85/40 | 80/80 |

Mean Particle Size: D[4,3] of the capsules after dispersion in volatile silicone (cyclopentadimethicone) was obtained using a Malvern Mastersizer 2000, the following parameters.

- RI of Dispersant = 1.397
- RI of capsule E1 = 1.430
- RI of capsule E2 = 1.530
- Dispersion module mixer speed = 2100rpm.
- Result calculation model = General purpose.
- Calculation sensitivity = Normal.
- Particle shape = Spherical

Shell Thickness: Measured by SEM on encaps with a particle size specified. For non-spherical encaps, the thickness was measured at or close to the minimum encapsulate diameter. Shell Thickness (Calculated): Calculation assumed that capsules were spherical, with a single core and the shell and core had the same density.
DR is the ratio of av. particle diameter : measured shell thickness.

Examples 1 and 2

In these Examples, the effectiveness of an encapsulated fragrance product was compared in the same anhydrous antiperspirant composition with a non-encapsulated fragrance having similar fragrance.
The effectiveness was determined in the following test in which 24 - 26 panelists self-applied approximately 0.3g example stick product (not claimed) to either the left or right armpit and comparison product to the other, with overall left-right randomized balance or an approximately 2 second spray. The test encapsulated floral-green fragrances E1 and E2 were compared with control formulations that contained either a fruity-floral (Bm) or a floral aldehydic (Cn) non-encapsulated fragrance. The four various combinations of encapsulated and non-encapsulated fragrances E1-Cn, E1-Bm, E2-Cn and E2-Bm are specified in the Tables below.
After application of the antiperspirant formulations, the users put on their normal clothing and the intensity of the odour was assessed at 2 hourly intervals on a scale of perception increasing from 0 to 10. The scores were averaged and that for the non-encapsulated sample deducted from that for the encapsulated sample. Three scores were measured, namely intensity of the fragrance itself, the intensity detected through the clothing and finally the intensity of any malodour. The results using a representative stick formulation (not claimed) are summarized in Table 2 below, in a representative aerosol formulation in Table 3.
The tested formulations were as follows:-

Table 2

| | Stick | Aerosol Base |
|---|---|---|
| Ingredient | % by weight | |
| Cyclomethicone | balance | balance |

(continued)

|  | Stick | | | | Aerosol Base | | |
|---|---|---|---|---|---|---|---|
| Ingredient | % by weight | | | | | | |
| Ester oil | 15.0 | | | | | | |
| Ether Oil | 9.5 | | | | 23.1 | | |
| Dimethiconol in cyclomethicone | | | | | 3.8 | | |
| Stearyl alcohol | 18.0 | | | | | | |
| Castorwax | 3.5 | | | | | | |
| Polyethylene wax | 1.0 | | | | | | |
| Suspending Aid | | | | | 3.8 | | |
| Swelling Aid | | | | | 0.1 | | |
| AZAG | 24.00 | | | | | | |
| AACH | | | | | 38.5 | | |
| AP cogellant | | | | | 3.8 | | |
| Preservative | 0.05 | | | | | | |
| Fragrance | E1 | E2 | Cn | Bm | E1 | E2 | Cn |
| amount | 1.5 | 0.7 | 1.2 | 1.5 | 4.6 | 2.3 | 4.6 |

In the stick (not claimed) and aerosol compositions, the proportion of fragrance employed from E1 and E2 was approximately the same, about 0.6% in the stick and about 1.8% in the aerosol base, and the proportion of each non-encapsulated fragrance was substantially higher at the outset of the comparisons.

Table 3 Stick Results (Example 1)

| Assessment time Hrs) | Difference in Intensity at assessment | | | | | |
|---|---|---|---|---|---|---|
| | Direct | | Through Clothing | | Malodour | |
| | Cn+E1 v Cn | Bm+E1 v Bm | Cn+E1 v Cn | Bm+E1 v Bm | Cn+E1 v Cn | Bm+E1 v Bm |
| 0 | 0.89 | 0.92 | 0.09 | 1.31 | n/d | n/d |
| 2 | 1.5 | 0.96 | 2.0 | 1.62 | -0.17 | n/d |
| 4 | 1.63 | 1.28 | 2.41 | 1.69 | -0.17 | n/d |
| 6 | 2.04 | 1.12 | 2.16 | 1.54 | -0.08 | n/d |
| 8 | 1.52 | 1.4 | 2.59 | 1.54 | -0.38 | n/d |
| 10 | 1.64 | 0.92 | 1.91 | 1.73 | -0.46 | n/d |
| 12 | 1.5 | 0.86 | 1.58 | 1.54 | -0.50 | n/d |
| 14 | 1.04 | 0.68 | 1.37 | 0.93 | -0.17 | n/d |
| | | | | | | |
| Assessment time (Hrs) | Difference in Intensity at assessment | | | | | |
| | Direct | | Through Clothing | | Malodour | |
| | Cn+E2 v Cn | Bm+E2 v Bm | Cn+E2 v Cn | Bm+E2 v Bm | Cn+E2 v Cn | Bm+E2 v Bm |
| 0 | 0.09 | 1.31 | 0.71 | 0.81 | n/d | n/d |
| 2 | 2.04 | 1.62 | 1.54 | 1.19 | -0.12 | 0 |
| 4 | 2.41 | 1.69 | 1.29 | 1.84 | -0.2 | -0.08 |

(continued)

| Assessment time (Hrs) | Difference in Intensity at assessment | | | | | |
|---|---|---|---|---|---|---|
| | Direct | | Through Clothing | | Malodour | |
| | Cn+E2 v Cn | Bm+E2 v Bm | Cn+E2 v Cn | Bm+E2 v Bm | Cn+E2 v Cn | Bm+E2 v Bm |
| 6 | 2.16 | 1.54 | 1.54 | 0.89 | -0.42 | -0.13 |
| 8 | 2.59 | 1.54 | 1.63 | 1.84 | -0.46 | -0.27 |
| 10 | 1. 90 | 1.57 | 1.58 | 1.88 | -0.39 | -0.35 |
| 12 | 1.58 | 1.54 | 1.08 | 0.81 | -0.50 | -0.39 |
| 14 | 1.47 | 0.93 | 0.91 | 0.77 | -0.37 | -0.39 |

Table 4 Aerosol Results (Example 2)

| Assessment time (Hrs) | Difference in Intensity at assessment | | | | | |
|---|---|---|---|---|---|---|
| | Direct | | Through Clothing | | Malodour | |
| | Cn+E1 v Cn | Cn+E2 v Cn | Cn+E1 v Cn | Cn+E2 v Cn | Cn+E1 v Cn | Cn+E2 v Cn |
| 0 | 0.93 | 0.25 | 0.24 | 0.31 | n/d | n/d |
| 2 | 1.21 | 1.07 | 0.44 | 0.62 | -0.19 | 0.00 |
| 4 | 1.06 | 0.94 | 0.32 | 0.81 | -0.06 | 0.06 |
| 6 | 0.96 | 1.00 | 0.75 | 1.31 | 0.12 | -0.25 |
| 8 | 0.77 | 1.12 | 0.37 | 1.00 | -0.31 | -0.69 |
| 10 | 0.29 | 0.75 | 0.44 | 0.75 | -0.25 | -0.81 |
| 12 | 0.31 | 0.62 | 0.31 | 0.75 | -0.13 | -0.50 |

From Tables 3 and 4, it is apparent that a greater intensity of the fragrance was perceived from the encapsulated fragrance compared with the non-encapsulated alternative throughout the period of the trial, irrespective of whether was assessed through clothing or directly, i.e. not through clothing. In addition, when judging the presence of malodours, the panelists consistently generated negative differences, once a long enough period had elapsed for malodours to be generated in situ, showing once again the effectiveness of the encapsulated sample at masking malodour. The time span of the test represented a substantial fraction of the waking hours for many consumers.

Example 4

Clinical trials were conducted to demonstrate the difference in malodour suppression between encapsulated (test) and non-encapsulated (control) fragrance applied from a stick composition (not claimed). The formulations employed in Example 3 were the same as those employed in Example 1.

In this Example, test and control product was applied daily to the underarm of panelists (0.3g +/- 0.03g) and the panelist carried out normal daily activities until after 5 or 24 hours, the effectiveness of the fragrance was assessed by the trained assessor rubbing the underarm gently with latex-gloved fingers (10 strokes). After 2 minutes, the malodour was assessed, but on a scale of from 0 to 5. This was repeated on 4 days, with the panelist instructed not to wash the underarm or apply any other antiperspirant or deodorant during the trial.

Table 7 Stick (not claimed)

| Fragrance Comparison | Assessment before or after shear | Assessment after application (hours) | Odour Score |
|---|---|---|---|
| Cn+E1 v Cn | Before | 24 | -0.07 |
| Cn+E1 v Cn | After | 24 | -0.10 |
| Cn+E2 v Cn | Before | 24 | -0.12 |

(continued)

| Fragrance Comparison | Assessment before or after shear | Assessment after application (hours) | Odour Score |
|---|---|---|---|
| Cn+E2 v Cn | After | 24 | -0.18 |
| Bm+E1 v Bm | Before | 5 | -0.26 |
| Bm+E1 v Bm | After | 5 | -0.14 |
| Bm+E1 v Bm | Before | 24 | -0.10 |
| Bm+E1 v Bm | After | 24 | -0.11 |
| Bm+E2 v Bm | Before | 5 | -0.36 |
| Bm+E2 v Bm | After | 5 | -0.44 |
| Bm+E2 v Bm | Before | 24 | -0.14 |
| Bm+E2 v Bm | After | 24 | -0.14 |

Table 8 Aerosol

| Fragrance Comparison | Assessment before or after shear | Assessment after application (hours) | Odour change |
|---|---|---|---|
| Cn+E1 v Cn | Before | 5 | -0.04 |
| Cn+E1 v Cn | After | 5 | -0.03 |
| Cn+E1 v Cn | Before | 24 | -0.01 |
| Cn+E1 v Cn | After | 24 | -0.10 |
| Cn+E2 v Cn | Before | 5 | -0.10 |
| Cn+E2 v Cn | After | 5 | -0.17 |
| Cn+E2 v Cn | Before | 24 | -0.04 |
| Cn+E2 v Cn | After | 24 | -0.12 |

In Tables 7 and 8, results with a -v prefix show a reduction in malodour score relative to the control.

The results summarised in Tables 7 and 8 consistently show that users assessed that the compositions employing the encapsulated fragrances E1 and E2 reduced malodour to a greater extent than the non-encapsulated fragrances over an extended period time, be it 5 or 24 hours after application.

Example 5 and Comparisons A and B

The fragrance intensity was compared initially and after 7 hours for three compositions, made identically in the manner identified below, comparison A containing a full fragrance, comparison B also containing a fragrance accord and Example 5 also containing the same fragrance accord, but encapsulated in E2 in accordance with the instant invention.

Table 9

|  | Comp A | Comp B | Ex 5 (not claimed) |
|---|---|---|---|
| Ingredient | % by weight | | |
| cyclomethicone | balance | balance | balance |
| Ester oil 1 | 15 | 15 | 15 |
| Ether oil | 9.5 | 9.5 | 9.5 |
| Stearyl Alcohol | 18 | 18 | 18 |
| Ester Wax 1 | 3.5 | 3.5 | 3.5 |
| Hydrocarbon Polymer | 1 | 1 | 1 |

(continued)

|  | Comp A | Comp B | Ex 5 (not claimed) |
|---|---|---|---|
| Ingredient | % by weight | | |
| AZAG | 24 | 24 | 24 |
| Parfum | 1 | 1 | 1 |
| Fragrance Accord | - | 0.595 | - |
| Encap fragrance accord | - | - | 0.7 |
| Preservative | 0.05 | 0.05 | 0.05 |
| Intensity Assessment |  |  |  |
| 0 hrs | 4.7 | 6.2 | 5.8 |
| 7 hrs, no rubbing | 1.1 | 1.6 | 1.2 |
| 7 hrs after rubbing | 1.4 | 1.6 | 3.6 |

The fragrance intensity was tested by the following general method:-

1. weighed amounts of each of the formulations was deposited by wiping a freshly cut surface of the stick compositions across separate coded sheets of plastic film (average 0.80 , 0.77 or 0.77g respectively).(the rough side of clear document wallets)

2. The fragrance intensity for the deposits as assessed by a panel of 10 females aged between 18 and 54 against the scale given below at time = 0hrs.

3. the plastic films were stored in an oven at 37°C.

4. Each plastic film was removed from the oven after 5 hours, rubbed once with a gloved finger to mimic in-wear rubbing and then returned to the oven for one hour.

5. Each plastic sheet was allowed to equilibrate to RT for 1 hour.

6. The fragrance intensity of each was assessed without rubbing at time = 7hrs.

7. Finally, each product was rubbed as at 5 hours but in a different place, and fragrance intensity assessed again.

Product fragrance intensity was ranked on a 0 to 10 scale.

| Score | Descriptor |
|---|---|
| 0 | No Perfume |
| 2 | Slight Perfume |
| 4 | Definite Perfume |
| 6 | Moderate Perfume |
| 8 | Strong Perfume |
| 10 | Intense Perfume |

The average of 10 assessment scores for each composition is shown in Table 8 above. The encapsulated fragrance retained its ability to yield detectable fragrance when subjected to rubbing much better than either its own fragrance accord or a commercial fragrance that was not so encapsulated, when subjected to storage at an elevated temperature.

Other anhydrous formulations containing an encapsulated fragrance according to the instant invention.

Ingredients for the preparation of the products employed in Examples 1 to 18 respectively.

Table 10

| Ingredient | Name or Trade Name | Supplier |
|---|---|---|
| Cyclomethicone [1] | DC 245 | Dow Corning Inc |
| Ester oil 1 [2] | C12-15 alkyl benzoate Finsolv TN | Finetex |
| Ester oil 2 [3] | Isopropyl myristate Estol 1512 | Uniqema |
| Ester Oil 3 | 2-phenyl ethyl benzoate Finsolv SUN | Finetex |
| Ether Oil | INCI PPG-14-butyl ether Fluid AP | Ucon Inc |
| Dimethicone | Dow Corning Fluid 200 (350 cSt) | Dow Corning Inc |
| Branched alcohol [4] | Isostearyl alcohol Prisorine 3515 | Uniqema |
| Dimethiconol in cyclomethicone | DC1501 | Dow Corning Inc |
| Stearyl alcohol [5] | Lanette C18 | Cognis |
| Ester wax 1 [6] | Castor wax Castorwax MP80 | CasChem Inc |
| Ester wax 2 [7] | Alkyl stearate behenate Kester Wax 82N | Koster Keunen |
| Ester wax 3 | Triglyceride wax Synchrowax HGL-C | Croda Ltd |
| Hydrocarbon wax 1 | Polyethylene Performalene 400 | New Phase Technologies (Baker Petrolite) |
| Hydrocarbon wax 2 | Paraffin wax SP173P, | Strahl & Pitsch Inc |
| Hydrocarbon Polymer | Styrene-ethylene/ butylene-styrene Block Copolymer Kraton G1650E | Kraton Polymers |
| SMGA 1 | N-(2-ethyl hexanoyl)-L-glutamic acid di-n-butylamide GA-01 | Ajinomoto |
| SMGA 2 | N-lauroyl-L-glutamic acid di-n-butylamide GP1 | Ajinomoto |
| SMGA 3 | 12-hydroxystearic acid | CasChem |
| Silicone Elastomer | 10% w/w in cyclomethicone DC9040 | Dow Corning Inc |
| Fumed silica | fumed silica Cab-o-sil | Cabot |
| Layered Clay | treated hectorite Bentone 38 | Rheox Inc |
| Swelling Aid | Propylene carbonate | |
| ACH | Aluminium chlorohydrate Micro Dry | Reheis Inc |
| AACH | Activated aluminium chlorohydrate A296 | B K Giulini GmbH |
| AZAG | Aluminium zirconium tatrachlorohydrex-Gly Reach 908 | Reheis Inc |
| Polymer AP cogellant | PVM/MA Copolymer Gantraz S95S | International Speciality Products |
| Preservative | Butylhydroxytoluene Tenox BHT | Eastman Chemicals |
| E1 | As described above | |
| E2 | As described above | |
| ES3 | Starch encapsulate | Givaudan |
| Free Fragrance | Ex Fragrance House | |

(continued)

| Ingredient | Name or Trade Name | Supplier |
|---|---|---|
| Propellant | Propane, butane and isobutane CAP40 | Calor Gas Ltd. |

Foot notes [1] DC245 can be replaced wholly or partly by DC246, or DC345™
Finsoln TN can be replaced wholly or partly by Finsolv TPP™
[3] Estol 1512 can be replaced wholly or partly by Estol 1517™
[4] Prisorine 3515 can be replaced wholly or partly by Eutanol G16™, (Cognis)
[5] Lanette 18 can be replaced partly (up to 50%) by Lanette 16™ and/or Lanette 22™
[6]Castorwax MP80 can be replaced wholly or partly by Castorwax MP90™.
[7]Kester Wax 62 can be replaced wholly or partly by Kester Wax 69H

Examples 1, 3, 5, and 6 to 11 (not claimed) and Comparisons A and B

In these Examples, stick products are made by filling a dispenser comprising a barrel oval in cross section having a base and an open top covered by a cap, a platform fitting snugly within the barrel at a position intermediate between the base and the top and advancement means for the platform mounted under the base, said means comprising a rotor wheel and an attached threaded spindle engaging a cooperating thread in the platform with a composition summarised in the Table below. The summarised stick compositions are made by the following general method.
The selected oil or oils are charged in the desired weight proportion into a vessel, the desired gellant or mixture of gellants in the desired weight proportion is introduced and the resultant mixture is agitated with an agitator of suitable power or by circulation through a recirculation loop, and heated until a temperature is reached at which the gellant or all the gellants have dissolved in the oils. For waxes that temperature is commonly in the range of from 75 to 90°C. For SMGAs, depending on the particular SMGA, that temperature is often from 90 to 120°C. Thereafter, the mixture is allowed to cool by 5 to 15°C and the desired weight proportion of particulates other than the encapsulated fragrance (including particularly the antiperspirant active) are introduced with continued agitation. The mixture is cooled or allowed to cool to a temperature of about 5 to 10°C above the normal setting temperature of the composition (which has been determined in a previous trial). Finally, with gentle agitation, the encapsulated fragrance and any non-encapsulated (free) fragrance are introduced and the still mobile composition is charged into the dispenser.

Stick formulations (not claimed)

Table 11

| Example No | 6 | 7 | 8 | 9 | 10 | 11 |
|---|---|---|---|---|---|---|
| Ingredient | Parts by weight | | | | | |
| Cyclomethicone | 34.0 | 26.0 | 47.5 | 25.0 | | 37.5 |
| Ester oil 1 | 6.0 | 15.0 | | 17.5 | | 10.0 |
| Ester oil 2 | 6.0 | | | | | |
| Ester Oil 3 | | | | | 53.15 | |
| Ether Oil | 10.0 | 9.5 | 15.0 | 15.5 | | 5.0 |
| Dimethicone | | | 5.0 | 1.0 | | |
| Branched alcohol | | | | | 11.45 | 14.0 |
| Stearyl alcohol | 15.5 | 18.0 | | | | |
| Ester wax 1 | 4.0 | 3.5 | | | | |
| Ester wax 2 | | | 10.0 | | | |
| Hydrocarbon wax 1 | | 1.0 | | 8.0 | | |
| Hydrocarbon wax 2 | | | | 6.0 | | |
| Hydrocarbon Polymer | | | | | 5.9 | |

(continued)

| Example No | 6 | 7 | 8 | 9 | 10 | 11 |
|---|---|---|---|---|---|---|
| Ingredient | Parts by weight | | | | | |
| SMGA 1 | | | | | 2.5 | |
| SMGA 2 | | | | | 2.5 | 2.5 |
| SMGA 3 | | | | | | 7.0 |
| SMGA 4 | | | | | | |
| ACH | 24.0 | | | | | |
| AACH | | | 20.0 | | | 22.0 |
| AZAG | | 24.0 | | 24.5 | 22.5 | |
| E1 | | 1.5 | 1.5 | | | 2.0 |
| E2 | 0.5 | | | 2.0 | 1.0 | |
| ES3 | | | | 0.5 | | |
| Free Fragrance | | 1.5 | 1.0 | | 1.5 | |

Examples 12 to 14 and 16 to 18

In Examples 12 and 13 (not claimed), soft solid or roll-on formulations are made. The soft solid formulations are charged into a dispenser having its top covered by a dome with narrow apertures. That made with a wax gellant are made by a similar process to that of the stick formulations, the amount being insufficient to produce a hard stick That made using a silica thickening agent comprises stirring a suspension of all the ingredients in a vessel at a temperature in the range of 25 to 50°C until an homogeneous suspension is obtained, and thereafter top filling it into the dispenser and placing the dome in the mouth.

In Example 14 (not claimed), a roll-on formulation is made by a similar method to Example 13, employing less thickener. In Example 15, the roll-on formulation is absorbed into a non-woven applicator cloth.

In Examples 2, 4, and 16 to 18, an aerosol product is made by the following general method. All the ingredients of the base composition (i.e. all except for the propellant) are blending in a vessel at ambient temperature until an homogenous mixture is obtained. Then the base composition is charged into a preformed aluminium can, a valve cup supporting a valve from which depends a dip tube is crimped into place, and propellant is charged into the can through the valve. Thereafter, an actuator is placed above the valve stem extending upwards from the valve.

Table 12

| Example No | 12 | 13 | 14 | 16 | 17 | 18 |
|---|---|---|---|---|---|---|
| Ingredient | Parts by weight | | | | | |
| Cyclomethicone | 32.5 | 32.0 | 54.5 | 7.0 | 2.79 | 5.6 |
| Ester oil 1 | 14.0 | 10.0 | 15.0 | 5.0 | | 1.0 |
| Ester oil 2 | 7.5 | | | 2.3 | | |
| Ester Oil 3 | | | | | | |
| Ether Oil | | 5.0 | 10.0 | | 3.0 | |
| Dimethicone | 8.0 | 7.0 | 2.0 | 3.0 | | |
| Ester wax 1 | | | | | | |
| Ester wax 3 | 3.25 | | | | | |
| Hydrocarbon wax 2 | 3.25 | | | | | |
| Silicone elastomer | 4.0 | | | | | |
| Fumed silica | | 5.0 | 1.5 | 0.4 | | |

(continued)

| Example No | 12 | 13 | 14 | 16 | 17 | 18 |
|---|---|---|---|---|---|---|
| Ingredient | Parts by weight | | | | | |
| Layered Clay | | | | 1.25 | 0.5 | 0.5 |
| Swelling Aid | | | | 0.05 | 0.01 | |
| ACH | | | 15.0 | 10.0 | | |
| AACH | | 12.0 | | | 5.0 | 5.0 |
| AZAG | 25.0 | 12.0 | | | | |
| Propellant | | | | 70.0 | 87.0 | 87.0 |
| E1 | 1.5 | | 1.0 | 0.5 | 0.6 | |
| E2 | | 1.5 | | | | 0.3 |
| ES3 | 0.5 | 0.5 | | | 0.1 | |
| Free Fragrance | 0.5 | | 1.0 | 0.5 | 1.0 | 0.6 |

**Claims**

1.  An anhydrous antiperspirant composition **characterised by** the absence of an aqueous phase comprising
    a particulate antiperspirant active,
    a dry particulate shear sensitive encapsulated perfume,
    a liquid carrier for the particulate antiperspirant active and shear sensitive encapsulated perfume comprising at least one water-immiscible oil in which the particulate encapsulated perfume comprises capsules having a shell of a cross-linked gelatin coacervate,
    a volume average particle diameter in the range of 25 to 70 $\mu$m,
    a shell having a measured thickness in the range of from 0.25 to 9 $\mu$m
    a ratio of the shell thickness to the average particle diameter in the range of from 1:5 to 1:120 and a Hysitron hardness, measured in a Hysitron Triboindenter fitted with a Berkovich tip and programmed to perform an indent by compressing a sample with an initial contact force of 75 $\mu$N, for 10 seconds, followed by a position hold stage for 1 second and a decompression stage for 10 seconds, in the range of from 1.5 MPa to 50 MPa,
    blended with a propellant for application from a pressurised aerosol dispenser.

2.  A composition according to claim 1 in which the coacervate is obtained by contacting gelatin with either

    - gum Arabic or a charged carboxymethyl cellulose at a pH of below 5.

3.  A composition according to claim 1 or 2 in which the coacervate is cross-linked with glutaraldehyde.

4.  A composition according to any preceding claim in which the capsules have a particle size D[4,3] in the range of from 40 to 60$\mu$m.

5.  A composition according to any preceding claim in which the capsules have a measured shell thickness in the range of up to 2.5$\mu$m.

6.  A composition according to any preceding claim in which the capsules have a water content of less than 5%.

7.  A composition according to any preceding claim which contains from 0.1 to 4% by weight of the capsules.

8.  A composition according to any preceding claim which additionally contains non-encapsulated fragrance.

9.  A composition according to any preceding claim which is free from ethanol.

**Patentansprüche**

1. Wasserfreie schweißhemmende Zusammensetzung, **gekennzeichnet durch** die Abwesenheit einer wässrigen Phase, umfassend

   einen teilchenförmigen schweißhemmenden Wirkstoff,

   einen trockenen teilchenförmigen, scherungsempfindlichen eingekapselten Duftstoff,

   einen flüssigen Träger für den teilchenförmigen schweißhemmenden Wirkstoff und den scherungsempfindlichen eingekapselten Duftstoff,

   umfassend mindestens ein mit Wasser nicht mischbares Öl,

   in welchem der teilchenförmige eingekapselte Duftstoff Kapseln umfasst mit einer Hülle eines vernetzten Gelatinekoazervats,

   einem volumengemittelten Partikeldurchmesser in dem Bereich von 25 bis 70 μm, einer Hülle mit einer gemessenen Dicke in dem Bereich von 0,25 bis 9 μm,

   einem Verhältnis der Hüllendicke zu dem durchschnittlichen Partikeldurchmesser in dem Bereich von 1:5 bis 1:120

   und einer Hysitron-Härte, gemessen in einem Hysitron-Triboindenter, ausgerüstet mit einer Berkovitch-Spitze und programmiert, um ein Eindrücken **durch** Komprimieren einer Probe mit einer anfänglichen Kontaktkraft von 75 μN, während 10 Sekunden, gefolgt von einer Positionshaltestufe für 1 Sekunde und einer Dekompressionsstufe für 10 Sekunden, in dem Bereich von 1,5 MPa bis 50 MPa, zu vollziehen,

   gemischt mit einem Treibmittel zur Anwendung aus einem druckbeaufschlagten Aerosolspender.

2. Zusammensetzung nach Anspruch 1, in welcher das Koazervat durch Inkontaktbringen von Gelatine mit entweder Gummi arabicum oder einer geladenen Carboxymethylcellulose bei einem pH unter 5.

3. Zusammensetzung nach Anspruch 1 oder 2, in welcher das Koazervat mit Glutaraldehyd vernetzt ist.

4. Zusammensetzung nach irgendeinem vorhergehenden Anspruch, in welcher die Kapseln eine Partikelgröße D[4,3] in dem Bereich von 40 bis 60 μm aufweisen.

5. Zusammensetzung nach irgendeinem vorhergehenden Anspruch, in welcher die Kapseln eine gemessene Hüllendicke in dem Bereich bis zu 2,5 μm aufweisen.

6. Zusammensetzung nach irgendeinem vorhergehenden Anspruch, in welcher die Kapseln einen Wassergehalt von weniger als 5% aufweisen.

7. Zusammensetzung nach irgendeinem vorhergehenden Anspruch, welche 0,1 bis 4 Gewichts-% der Kapseln enthält.

8. Zusammensetzung nach irgendeinem vorhergehenden Anspruch, die zusätzlich nicht-eingekapselte Aromen enthält.

9. Zusammensetzung nach irgendeinem vorhergehenden Anspruch, welche frei von Alkohol ist.


**Revendications**

1. Composition d'antiperspirant anhydre **caractérisée par** l'absence d'une phase aqueuse comprenant

   un actif d'antiperspirant particulaire,

   un parfum encapsulé sensible au cisaillement particulaire sec, un support liquide pour l'actif d'antiperspirant particulaire et le parfum encapsulé sensible au cisaillement comprenant au moins une huile immiscible à l'eau dans laquelle le parfum encapsulé particulaire comprend des capsules présentant une enveloppe d'un coacervat de gélatine réticulée,

   un diamètre moyen de particules en volume dans l'intervalle de 25 à 70 μm,

   une enveloppe présentant une épaisseur mesurée dans l'intervalle de 0,25 à 9 μm

   un rapport de l'épaisseur d'enveloppe au diamètre moyen de particule dans l'intervalle de 1:5 à 1:120

   et une dureté Hysitron, mesurée dans un Triboindenter Hysitron équipé d'une extrémité Berkovich et programmé pour réaliser une encoche par compression d'un échantillon avec une force de contact initiale de 75 μN, pendant 10 secondes, puis par une étape de maintien de position pendant 1 seconde et une étape de décompression pendant 10 secondes, dans l'intervalle de 1,5 MPa à 50 MPa,

   en combinaison avec un propulseur pour une application à partir d'un distributeur d'aérosol pressurisé.

**2.** Composition selon la revendication 1, dans laquelle le coacervat est obtenu par mise en contact de gélatine avec soit de la gomme arabique soit de la carboxyméthylcellulose chargée à un pH inférieur à 5.

**3.** Composition selon la revendication 1 ou 2, dans laquelle le coacervat est réticulé avec du glutaraldéhyde.

**4.** Composition selon l'une quelconque des revendications précédentes, dans laquelle les capsules présentent une taille de particule D[4,3] dans l'intervalle de 40 à 60 $\mu$m.

**5.** Composition selon l'une quelconque des revendications précédentes, dans laquelle les capsules présentent une épaisseur d'enveloppe mesurée dans l'intervalle allant jusqu'à 2,5 $\mu$m.

**6.** Composition selon l'une quelconque des revendications précédentes, dans laquelle les capsules présentent une teneur en eau inférieure à 5 %.

**7.** Composition selon l'une quelconque des revendications précédentes qui contient de 0,1 à 4 % en masse des capsules.

**8.** Composition selon l'une quelconque des revendications précédentes qui contient de plus du parfum non-encapsulé.

**9.** Composition selon l'une quelconque des revendications précédentes qui est exempte d'éthanol.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US P6045835 A
- US 6045835 A
- WO 2006056096 A
- US 4278658 A
- EP 6739 A, Unilever NV
- US 3792068 A, Luedders
- US 6616921 B
- EP 0303461 A
- EP 1289484 A
- EP 1219547 A
- EP 1255682 A
- EP 1749759 A

**Non-patent literature cited in the description**

- Compilation of Odor and Taste Threshold Values Data. American Society for Testing and Materials, 1978